# EUROPEAN PATENT APPLICATION

(11) **EP 4 125 164 A1**
(43) Date of publication of application: **01.02.2023**
(21) Application number: 20926596.6
(22) Date of filing: 24.12.2020
(51) Int. Cl.: H01R 13/639, H01R 13/502, H01R 24/00

(54) **CONNECTION DEVICE, CONNECTOR HAVING SAME, AND HEAT EXCHANGE APPARATUS**

(30) Priority: 25.03.2020 CN 202010220312
(71) Applicant: Suzhou Microport Rehabtech (Group) Co., Ltd., Suzhou, Jiangsu 215000 (CN)
(72) Inventor: RUAN, Jiantao, Suzhou, Jiangsu 215000 (CN); LUO, Yi, Suzhou, Jiangsu 215000 (CN); DI, Pei, Suzhou, Jiangsu 215000 (CN)
(74) Representative: Lavoix
(86) International application number: PCT/CN2020/139173
(87) International publication number: WO 2021/190014

(57) **Abstract**

The present invention provides a connection device, as well as a connector and heat exchange device, both having the connection device. The connection device includes a plurality of first connectors, a casing, a pressing member and a fastening assembly. The plurality of the first connectors, the pressing member and the fastening assembly are all at least partially housed in the casing, and the pressing member has a pressing portion which is exposed out of the casing. The first connectors are adapted to mate with respective second connectors. The fastening assembly is configured to switch between a ready-to-fasten configuration and a fastening configuration and thereby change connection conditions between the first and second connectors. Firstly, the connection device of the present invention is overall simple in structure and space-saving. Secondly, connecting and fastening the male and female connectors using the pressing member and the fastening assembly allows fast mating and unmating to be achieved by convenient operations. Thirdly, fastening a plurality of connectors, such as multiple pairs of male and female connectors, using the single fastening assembly results in additional significant decreases in manufacturing cost, provides the user with increased convenience of use and enables easier operations.

## Description

### TECHNICAL FIELD

The present invention relates to the techncial field of connectors and, in particular, to a connection device for a heat exchange apparatus, a connector, and a heat exchange apparatus.

### BACKGROUND

In a heat exchange device, a connection is usually established with a male connector and a female connector. For example, in a cold and hot compress pressurization device, three male connectors and three female connectors are used to create any combination of three water/gas channels. However, conventionally, the channels are individually established and eliminated with three independently controllable pairs of male and female connectors. This design not only leads to high manufacturing cost of the male and female connectors but requires complicated operations of a user. For example, the user has to perform multiple different operations in order to mate/unmate or fasten the connectors. These multiple operations easily cause damage to the male and female connectors.

Therefore, it is desirable to design a connection device, which has a simple structure, can be manufactured at low cost, is easy to use and allows fastening of multiple connectors.

### SUMMARY OF THE INVENTION

It is an object of the present invention to provide such a connection device that has a simple structure, can be manufactured at low cost, is easy to use and allows fastening of multiple connectors, as well as a connector and a heat exchange device both having the connection device.

To this end, the present invention provides a connection device comprising a plurality of first connectors, a casing, a pressing member and a fastening assembly,
the plurality of the first connectors, the pressing member and the fastening assembly all at least partially housed in the casing, the pressing member having a pressing portion which is exposed out of the casing and configured to drive the fastening assembly, the first connectors configured to connect with respective second connectors,
the fastening assembly configured to switch between a ready-to-fasten configuration and a fastening configuration and thereby change connection conditions between the first and second connectors.

Optionally, the fastening assembly may comprise a fastener, a first elastic member, a push pin and a second elastic member, the fastener movably arranged at one of the first connectors, the first elastic member abutting, at its opposite ends, against the fastener and the specific first connector and configured to drive the fastener to move up and down, the second elastic member abutting, at its opposite ends, against the push pin and the first connector and configured to drive the push pin to move horizontally.

Optionally, the ready-to-fasten configuration of the connection device may result from pressing the pressing portion to cause the fastener to move over an end surface of the first connector from a first position to a second position where the fastener is engaged by the push pin,
wherein the fastening configuration of the connection device results from inserting a respective one of the second connectors into the first connector so that the second connector abuts against the push pin and disengages the fastener from the push pin, as a result of which the fastener fastens the second connector.

Optionally, the fastener may be a bent plate comprising a first plate portion and a second plate portion extending from an end of the first plate portion, the first plate portion connected to the pressing member and the first elastic member in such a manner that the first elastic member abuts, at its opposite ends, against the first plate portion and the first connector, the second plate portion arranged at an end of the first connector.

Optionally, the second plate portion may defines first opening and a second opening, the first opening is configured for passage of the second connector therethrough, the second opening is configured for passage of the push pin therethrough, the first opening matches with a groove in the second connector, and the first opening is able to engage with the groove to fasten the second connector.

Optionally, the pressing member may define, at an end thereof, a receptacle pocket in which the first plate portion is inserted, and the pressing member may be, at the other end thereof, pivotally coupled to the casing.

Optionally, the first connector may be provided on the end surface with guide features in which the second plate portion is arranged so as to move up and down over the end surface of the first connector to change a configuration of the fastening assembly.

Optionally, the first connector may define a first accommodating recess, wherein an end of the first elastic member is received in the first accommodating recess and the other end thereof abuts against the fastener.

Optionally, the push pin may be cylindrical and comprise a first cylindrical section and a second cylindrical section, which are joined to each other, the first cylindrical section having a width that is less than a width of the second cylindrical section, the first cylindrical section configured to engage the fastener, the second cylindrical section configuerd to abut against the second elastic member.

Optionally, the first cylindrical section may define, around its middle, an annular groove, wherein the second opening comprises a first opening portion and a second opening portion, which are joined to each other, the first opening portion configured for passage of the first cylindrical section therethrough, the second opening portion configured to engage the annular groove.

Optionally, the first connector may define a second accommodating recess, wherein an end portion of the second elastic member is received in the second accommodating recess and the other end thereof abuts against the push pin.

Optionally, the casing may comprise an upper shell and a lower shell, the upper shell defining a through hole through which the pressing portion is exposed out of the casing.

Optionally, the casing may define a plurality of accommodating spaces for locating the respective first connectors, wherein the single fastening assembly is disposed in correspondence with one of the accommodating spaces in such a manner the single fastening assembly can be manipulated to simultaneously change the connection conditions between the first and second connectors.

Optionally, the fastening assembly may be disposed in correspondence with a middle one of the accommodating spaces.

The present invention also provides a connector comprising second connectors and the connection device as defined above.

The present invention also provides a heat exchange device comprising the connector as defined above and at least one of a fluidic connection pipe configured to be connected to the connector, a heat exchange and pressurization bag and a heat exchange and pressurization apparatus.

The connection device, connector and heat exchange device having the same according to the present invention have the following advantages over the prior art:
(1) The connection device of the present invention includes a plurality of first connectors, a casing, a pressing member and a fastening assembly. The plurality of the first connectors, the pressing member and the fastening assembly are all at least partially housed in the casing, and the pressing member has a pressing portion which is exposed out of the casing and is adapted to drive the fastening assembly. The fastening assembly is configured to switch between a ready-to-fasten configuration and a fastening configuration and thereby change connection conditions between the first and second connectors. Firstly, in the connection device of the present invention, a fastening structure is provided on the basis of elastic members for conventional male and female connectors, dispensing with the need for additionally designed elastic members. This results in reductions in cost, a simple structure and space savings in the connection device. Secondly, connecting and fastening the male and female connectors using the pressing member and the fastening assembly allows fast mating and unmating of the male and female connectors to be achieved by convenient operations. Thirdly, fastening a plurality of connectors, such as multiple pairs of male and female connectors, using the single fastening assembly results in additional significant decreases in manufacturing cost, provides the user with increased convenience of use and enables easier operations.
(2) Since the connector and heat exchange device of the present invention both include the connection device as defined above, they both have all the advantages of the connection device as discussed above.

### BRIEF DESCRIPTION OF THE DRAWINGS

In order to describe embodiments of the present invention or solutions of the prior art more clearly, a brief description of the drawings that are referenced in the description of the embodiments or solutions is set forth below. Apparently, these drawings show only some embodiments of the present invention, and one of ordinary skill in the art can obtain from them other drawings showing different embodiments without paying any creative effort. In these drawings:
Fig. 1 is an exploded view of a connection device according to an embodiment of the present invention;
Fig. 2 is a schematic diagram showing the connection device in a fastening configuration in which it is connected to male connectors;
Fig. 3 is a schematic diagram showing the structure of an upper shell of Fig. 1;
Fig. 4 is a schematic diagram showing the structure of a lower shell of Fig. 1;
Fig. 5 is a schematic diagram showing the structure of a female connector;
Fig. 6a is a schematic diagram showing a male connector being connected to a fastener in a ready-to-fasten configuration;
Fig. 6b is a schematic diagram showing the male connector being connected to the fastener in a fastening configuration;
Fig. 7 is a schematic diagram showing the structure of a pressing member of Fig. 1;
Fig. 8 is a schematic diagram showing the structure of the fastener of Fig. 1;
Fig. 9 is a schematic diagram showing the structure of a push pin of Fig. 1;
Fig. 10 is a schematic diagram showing a female connector being assembled with the lower shell of Fig. 1;
Fig. 11 is a cross-sectional view of Fig. 10 taken along line A-A;
Fig. 12 is a cross-sectional view of the connection device of Fig. 1, in which the male connectors have not been inserted into the female connectors yet and the connection device is in the ready-to-fasten configuration;
Fig. 13 is a cross-sectional view of the connection device of Fig. 1, in which the male connectors have been inserted into the female connectors and the connection device is in the fastening configuration; and
Fig. 14 is a schematic diagram showing the structure of a heat exchange device according to an embodiment of the present invention.

### List of Reference Numerals:

100: Casing; 110: Upper Shell; 111: Through Hole; 120: Lower Shell; 121: Shaft Seat;
200: Pressing Member; 210: Pressing Portion; 220: Receptacle Pocket; 230: Rotating Shaft;
300: Fastening Assembly; 310: Fastener; 311: First Plate Portion; 312: Second Plate Portion; 313: First Opening; 314: Second Opening; 3141: First Opening Portion; 3142: Second Opening Portion; 320: First Elastic Member; 330: Push Pin; 331: First Cylindrical Section; 332: Second Cylindrical Section; 333: Annular Groove; 334: First Side Wall; 340: Second Elastic Member;
400: Male Connector; 410: Groove; 401: Flange;
500: Female Connector; 510: First Accommodating Recess; 520: Second Accommodating Recess; 530: Guide Feature;
170: Connector; 21: Fluidic Connection Pipe; 20: Heat Exchange and Pressurization Bag; 10: Heat Exchange and Pressurization Apparatus; 191: Display Screen; 192: Handle.

### DETAILED DESCRIPTION

The present invention will be described in greater detail below with reference to the accompanying drawings and particular embodiments. From the following description, advantages and features of the present invention will become more apparent. Note that the drawings are provided in a very simplified form not necessarily drawn to exact scale for the only purpose of helping to explain the disclosed embodiments in a more convenient and clearer way. In order that objects, features and advantages of the present invention may become readily apparent, reference is made to the accompanying drawings. It should be noted that architectural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all architectural modifications, proportional variations or dimensional changes that do not affect the benefits and objects of the present invention are considered to fall within the scope of the teachings herein.

The present invention seeks primarily to provide a connection device, as well as a connector and a heat exchange device, both having the connection device, which solve the problems of a complicated design, high cost and inconvenience of use associated with conventional connection devices.

It is to be noted that all the male and female connectors, push pin and first and second elastic members in the present invention can be implemented as conventional standard parts. Therefore, the connection device of the present invention can be designed on the basis of conventional standard products. Through effective utilization of elastic members for conventional male and female connectors as a fastening structure, designing of additional elastic members is dispensed with, and significant reductions in cost and a simple and compact structure are achieved. In addition, in the connection device of the present invention, a single fastening assembly can be used to accomplish the fastening of a plurality of connectors, such as multiple pairs of male and female connectors. This results in additional significant decreases in manufacturing cost, provides a use with increased convenience of use and enables easier operations.

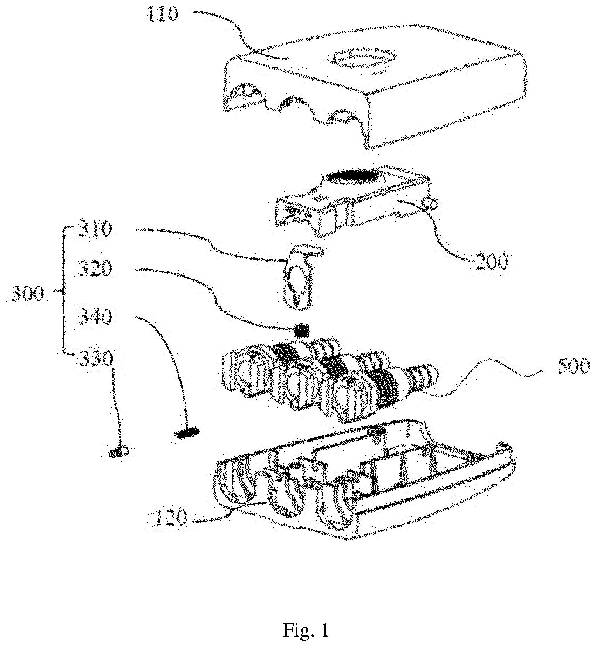

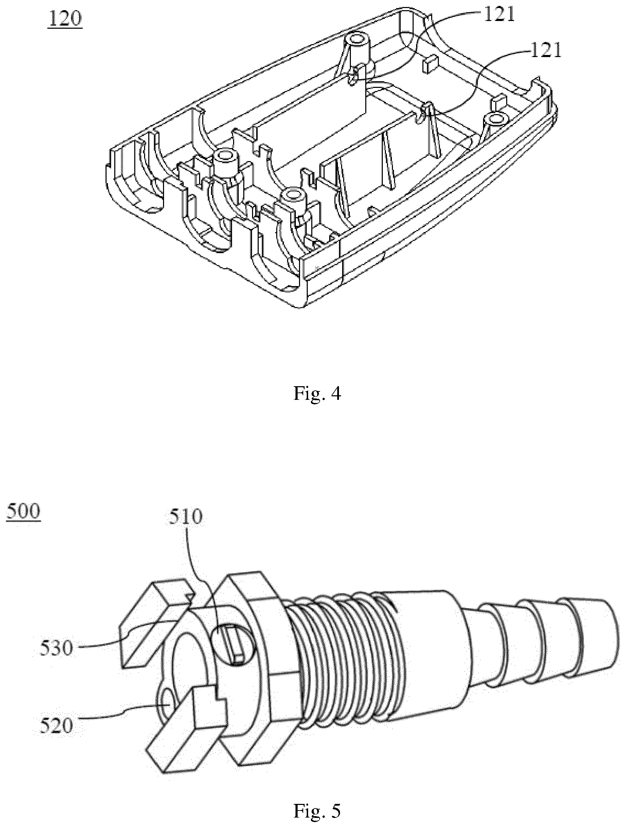

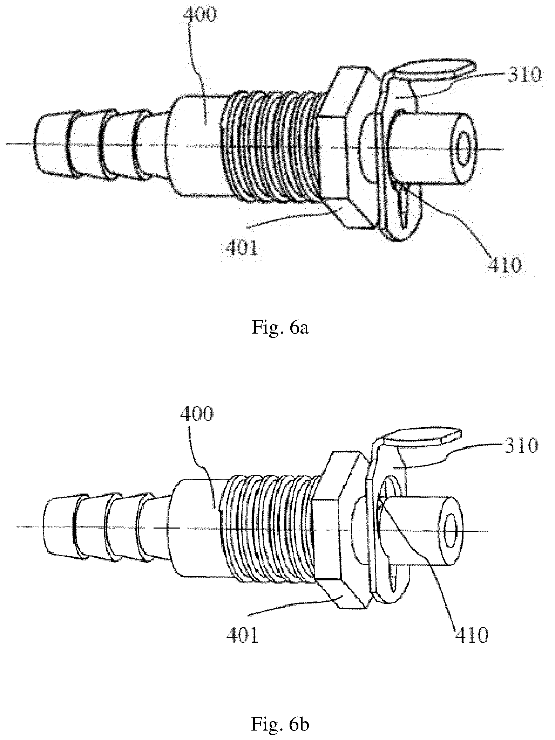

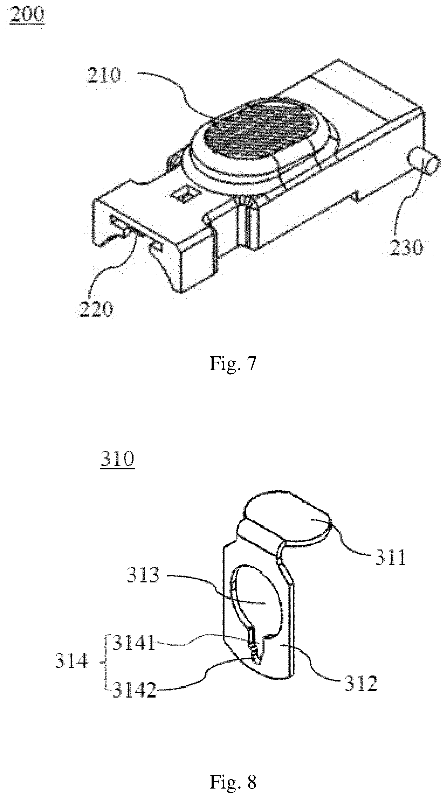

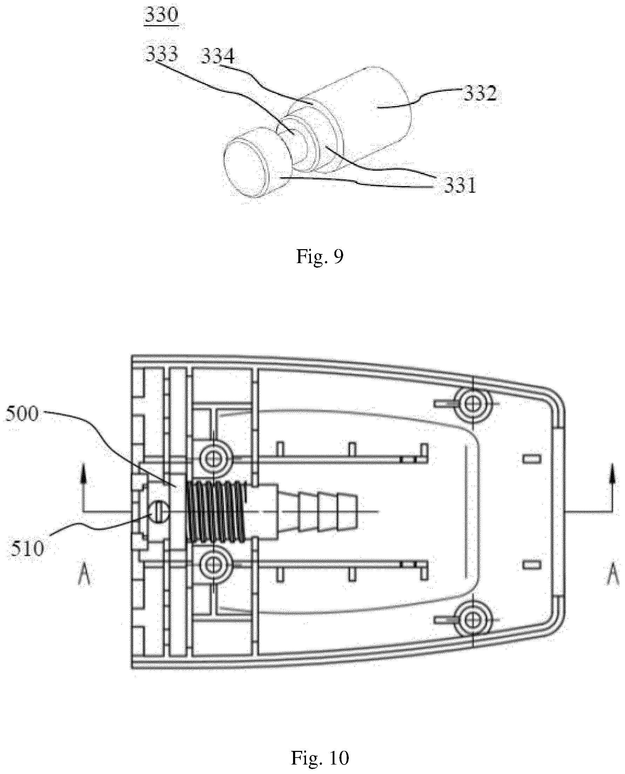

A connection device according to the present invention will be described below with reference to Figs. 1 to 14.

The connection device is adapted to connecting first and second connectors, which are, for example, male connectors 400 and female connectors 500. The connection device includes a plurality of female connectors 500, a casing 100, a pressing member 200 and a fastening assembly 300. The plurality of female connectors 500, the pressing member 200 and the fastening assembly 300 are each at least partially housed in the casing 100. The pressing member 200 further has a pressing portion 210, which is exposed out of the casing 100 and adapted to drive the fastening assembly 300. Specifically, the casing 100 includes an upper shell 110 and a lower shell 120. The upper shell defines a through hole 111, through which the pressing portion 210 is exposed out of the casing 100.

The fastening assembly 300 is configured to switch between a ready-to-fasten configuration and a fastening configuration and adapted to control connections of the plurality of female connectors 500 with the male connectors 400. Specifically, the fastening assembly 300 includes a fastener 310, a first elastic member 320, a push pin 330 and a second elastic member 340. The fastener 310 is movably disposed at any of the female connectors 500. The first elastic member 320 abuts, at its opposite ends, against the fastener 310 and the female connector 500, and is adapted to drive the fastener 310 to move up and down. The second elastic member 340 abuts, at its opposite ends, against the push pin 330 and the female connector 500, and is adapted to drive the push pin 330 to move horizontally.

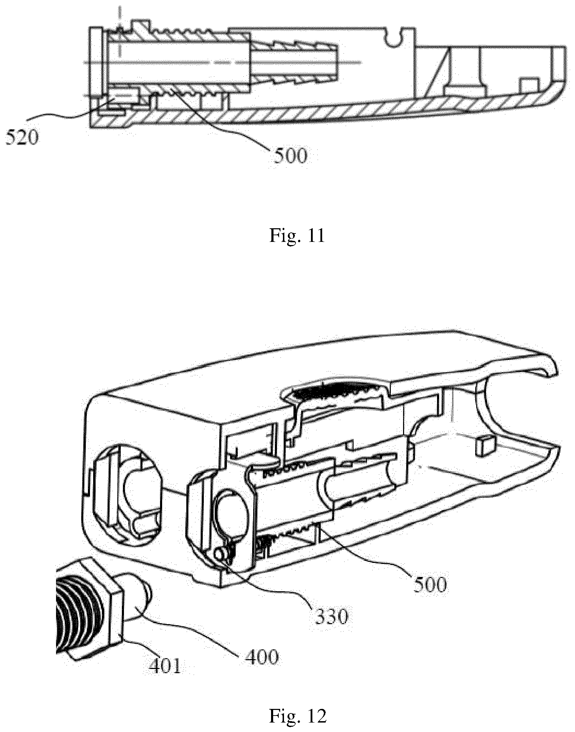

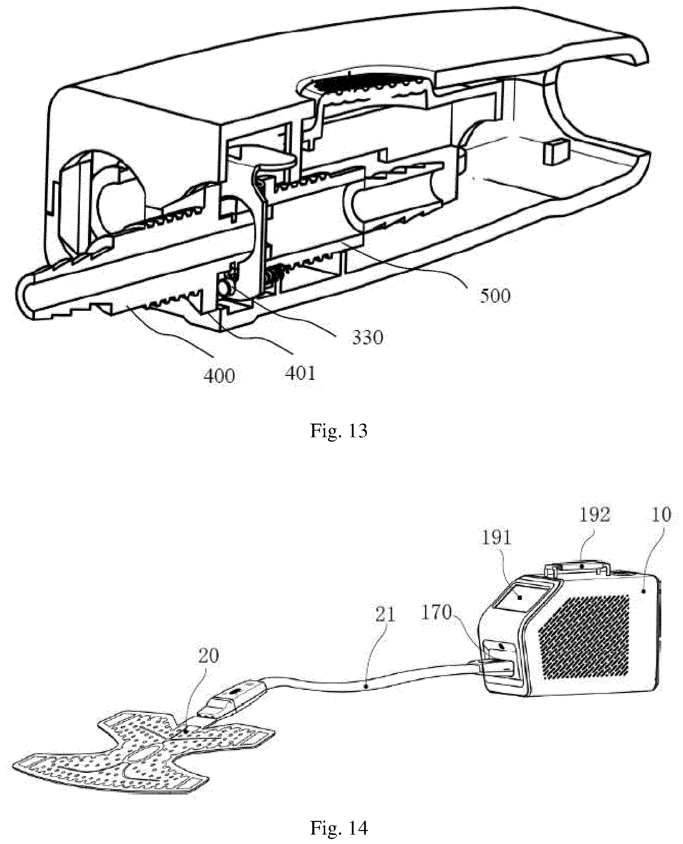

In operation, with the fastener 310 being at the first position, the pressing portion 210 is pressed to cause the fastener 310 to move across an end surface of the female connector 500 to a second position where it is engaged with the push pin 330. With this done, the connection device is in the ready-to-fasten configuration, as shown in Fig. 12. After that, the male connector 400 is inserted into the female connector 500 and concurrently pushes the push pin 330 against the female connector 500. As a result, the fastener 310 is disengaged so as to fasten the male connector 400, bringing the connection device into the fastening configuration, as shown in Fig. 13.

In order to enable quick connection and disconnection between the male connector 400 and the female connector 500, the connection device can have the following configurations: an initial configuration before the male connector 400 is engaged with the female connector 500 for the first time, in which the fastener 310 is maintained at the first position under the action of an elastic force from the first elastic member 320; the ready-to-fasten configuration, in which the connection device prepares itself for connection between the male connector 400 and the female connector 500, and which results from movement of the fastener 310 to the second position caused by pressing the pressing portion 210, where the push pin 330 engages the fastener 310 under the action of an elastic force from the second elastic member 340 and prevents the return of the fastener 310 back to the first position; and the subsequent fastening configuration resulting from insertion of the male connector 400 into the female connector 500, which leads to disengagement of the fastener from the push pin 330. The disengaged fastener 310 tends to return back to the first position under the action of the first elastic member 320, thereby fastening the male connector 400. It can be understood that, in order to disconnect the male connector 400 from the female connector 500, the pressing portion 210 can be again pressed to drive the fastener 310 again to the second position. This allows removal of the male connector 400 from the female connector 500. As a result, the push pin 330 moves externally under the action of an elastic force from the second elastic member and again engages and locks the fastener 310, switching the connection device back to the ready-to-fasten configuration.

In the present embodiment, the fastener 310 is a bent plate including a first plate portion 311 and a second plate portion 312 extending from one end of the first plate portion 311. The first plate portion 311 is connected to the pressing member 200 and the first elastic member 320, and the first elastic member 320 abuts, at its opposite ends, against the first plate portion 311 and the female connector 500. The second plate portion 312 is located at one end of the female connector 500. The pressing member 200 defines, at its one end, a receptacle pocket 220 into which the first plate portion 311 can be inserted. In this way, the pressing member 200 can be fixed to the fastener 310. The pressing member 200 is pivotally coupled at the other end to the casing 100. When the pressing portion 210 of the pressing member 200 is pressed, the pressing member 200 can pivot about a coupling feature at the other end, causing the fastener 310 to move from the first position to the second position. Specifically, a rotating shaft 230 is disposed at the other end of the pressing member 200, and shaft seats 121 are provided in the casing 100 (more precisely, on the lower shell 120). The pressing member 200 is pivotally coupled to the casing 100 by placing the rotating shaft 230 in the shaft seats 121.

Preferably, the first elastic member 320 is a spring, and the female connector 500 defines a first accommodating recess 510 or a locator hole. An end portion of the first elastic member 320 is received in the first accommodating recess 510, securely connecting the first elastic member 320 to the female connector 500. The other end of the first elastic member 320 abuts against the fastener 310. In particular, the other end of the first elastic member 320 may abut against the side of the first plate portion 311 of the fastener 310 that faces the female connector 500.

Further, the second plate portion 312 defines a first opening 313 and a second opening 314. The first opening 313 is sized to match the male connector to allow passage of the male connector therethrough. The second opening 314 is adapted to allow passage of the push pin 330 therethrough. The push pin 330 is cylindrical and includes a first cylindrical section 331 and a second cylindrical section 332 joined to the first cylindrical section 331. A width of the first cylindrical section 331 (e.g., a diameter of the circular cross-section of the first cylindrical section) is less than a width of the second cylindrical section 332 (e.g., a diameter of the circular cross-section of the second cylindrical section). A first portion of the first cylindrical section 331 at which it is joined to the second cylindrical section 332 can be inserted in the second opening 314, thereby engaging and locking the fastener 310. A second portion of the first cylindrical section 331 at a free end thereof is always maintained external to the fastener 310. A groove 333 is defined between the first and second portions. The second opening 314 is so sized as to allow passage of the first portion of the first cylindrical section 331 therethrough but disallow passage of the second cylindrical section 332 therethrough. With this design, when the fastener 310 reaches the second position, i.e., when it is moves downward to the second position under the action of the pressing portion 210, as a result of downward movement of the second opening 314 with the fastener 310, the push pin 330 will pass through the second opening 314 externally under a horizontal force exerted by the second elastic member 340 until a first side wall 334 of the push pin 330 comes into abutment against the second plate portion 312 of the fastener 310, thus locking the fastener 310 and preventing it from moving upward toward the original position.

Specifically, the second opening includes a first opening portion 3141 and a second opening portion 3142, which are joined to each other. The first opening portion 3141 is adapted for passage of the first portion of the first cylindrical section 331 therethrough, while the second opening portion 3142 is adapted to engage the annular groove 333. The second opening portion 3142 may be sized to match the annular groove 333 of the first cylindrical section 331 but disallow passage of the first cylindrical section 331 therethrough.

Preferably, the second elastic member 340 is a spring, and the female connector 500 defines a second accommodating recess 520. An end portion of the second elastic member 340 is received in the second accommodating recess 520, and the other end of the second elastic member 340 abuts against the push pin 330. In particular, the other end of the second elastic member 340 may abut against the second cylindrical section 332 of the push pin 330. Arranging the second elastic member 340 in the second accommodating recess 520 in the female connector 500 enables the second elastic member 340 to be securely connected to the female connector 500 and the push pin 330.

At first, the pressing portion 210 is pressed to cause the fastener 310 to move downward. Externally biased by the second elastic member 340, the push pin 330 drives the first cylindrical section 331 to pass through the first opening portion 3141 in the fastener 310 to bring the first side wall 334 into abutment against the second plate portion 312 of the fastener 310, as shown in Fig. 12. During insertion of the male connector 400 into the first opening 313, since the second portion of the first cylindrical section 331 is located external to the fastener 310 or in front of the first opening portion 3141, a flange 401 of the male connector 400 is brought, on an inner side thereof, into abutment against the second portion of the first cylindrical section 331 and then pushes the first cylindrical section 331 backward (i.e., toward the second elastic member 340) until the annular groove 333 moves into the first opening portion 3141. At this point, upward biased by the first elastic member 320, the fastener 310 moves upward back, bringing the annular groove 333 into the second opening portion 3142, as shown in Fig. 13. As a result, the fastener 310 is disengaged from the push pin 330 and then fastens the male connector 400. In order to release the male connector 400, the pressing portion 210 is again pressed to cause downward movement of the fastener 310 and hence of the first opening portion 3141. As a result, the second elastic member 340 externally biases the first cylindrical section 331, resulting in passage of the first portion of the first cylindrical section 331 through the first opening portion 3141 until the first side wall 334 comes into abutment against the second plate portion 312 of the fastener 310. At this point, the connection device is again in the ready-to-fasten configuration shown in Fig. 12.

In addition, in order to enable the fastener 310 to more firmly fasten the male connector 400, the male connector 400 may define a recess 410 that matches the second opening 314. In this case, after the fastener 310 is disengaged from the push pin 330, it is biased by the first elastic member 320 toward the first position, bringing the second opening 314 into engagement with the recess 410. As a result, the male connector 400 is fastened.

Further, the female connector 500 is provided on its end surface with a pair of guide features 530, which delimit a space together with the female connector 500, in which the second plate portion 312 can move up and down over the end surface of the female connector 500 to switch the fastening assembly between different configurations. Arranging the guide features 530 allows stable movement of the second plate portion 312. That is, it can reciprocate linearly within the guide features 530. This enables the fastener 310 and the female connector 500 to be more compact in structure.

The connection device of the present invention is adapted for quick connection between the male connectors 400 and the female connectors 500. The present invention is not limited to any particular numbers of the male connectors 400 and the female connectors 500. That is, the connection device may be adapted for connection of at least one pair of male 400 and female 500 connectors. The numbers of the connected male connectors 400 and female connectors 500 may be determined as actually required. In one embodiment, the connection device of the present invention is used in a cold and hot compress pressurization device for connection of three pairs of male 400 and female 500 connectors. Accordingly, the casing 100 defines accommodating spaces for the three female connectors 500 (not shown). The single fastening assembly 300 is disposed in correspondence with one of the accommodating spaces and may be manipulated to simultaneously adjust connections of the multiple female connectors 500 and male connectors 400. The three pairs of male 400 and female 500 connectors constitute three-channel connectors which can be mated or unmated to simultaneously establish or eliminate three channels. Therefore, integrating the three female connectors 500 by disposing them in the respective accommodating spaces in the casing 100 facilitates simultaneous establishment and elimination of the three channels. Arranging only the single fastening assembly 300 in one of the accommodating spaces rather than respective fastening assemblies 300 in the three accommodating spaces can avoid asynchronous operations of the fastening assemblies 300 during the mating or unmating process and avoid the risk of insufficient retention of the male connectors 400 and the female connectors 500. Moreover, the single fastening assembly 300 can ensure robustness of all the three channels. Preferably, the single fastening assembly 300 is arranged in correspondence with the middle accommodating space. This facilitates simultaneous fastening for the connectors of the three channels because fastening for the connectors of the middle channel allows better fastening for those of the remaining channels.

In summary, in the connection device of the present invention, a fastening structure is provided on the basis of elastic members for conventional male and female connectors, dispensing with the need for additional elastic members. This results in reductions in cost, a simple structure and space savings in the connection device. Moreover, connecting and fastening the male and female connectors using the pressing member and the fastening assembly allows fast mating and unmating of the male and female connectors to be achieved by convenient operations. Additionally, fastening a plurality of connectors, such as multiple pairs of male and female connectors, using the single fastening assembly results in additional significant decreases in manufacturing cost, provides the user with increased convenience of use and enables easier operations. In particular, when the connection device of the present invention is used in a three-channel connector of a cold and hot compress pressurization device, connectors for the three channels can be integrated in the casing of the connection device and synchronously manipulated with the fastening assembly, avoiding the risk of asynchronous fastening of the three channels.

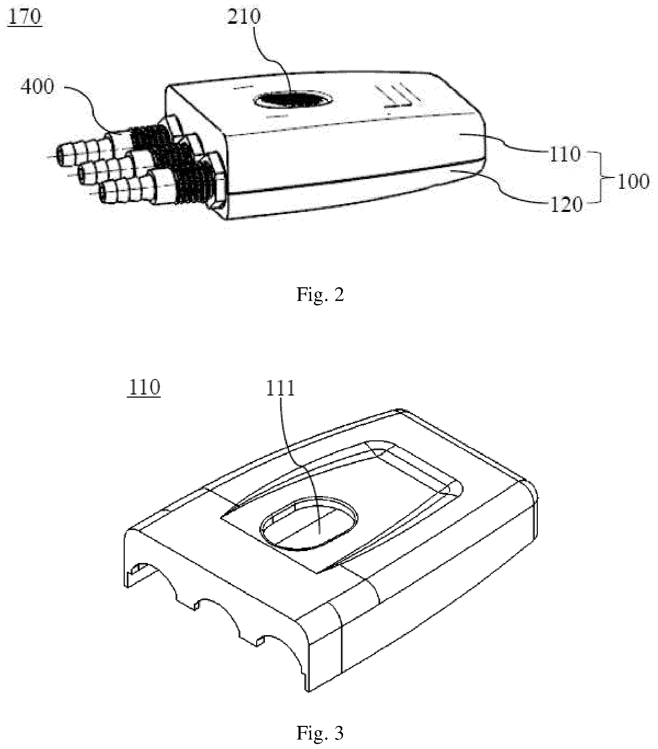

On the basis of an inventive concept similar to that described above, the present invention also provides a connector. As shown in Fig. 2, the connector 170 includes male connectors 400 and the connection device as defined above. As shown in Fig. 14, the present invention also provides a heat exchange device including the connector 170, a fluidic connection pipe 21, a heat exchange and pressurization bag 20 and a heat exchange and pressurization apparatus 10. This heat exchange device relies on the triplex connector 170 for water circulation and air pressurization. The heat exchange and pressurization apparatus 10 is equipped with a display screen 191 and a handle 192.

Since the connector and heat exchange device of the present invention both include the connection device as defined above, they both have all the advantages of the connection device as discussed above.

It is to be noted that, as used herein, relational terms such as first and second, etc., are only used to distinguish one entity or operation from another entity or operation, and do not necessarily require or imply these entities having such an order or sequence. Moreover, the terms "include," "including," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.

In the description herein, it would be appreciated that the orientational or positional relationships described by the terms "central", "longitudinal", "transverse", "length", "width", "thickness", "upper", "lower", "front", "rear", "left", "right", "vertical", "horizontal", "top", "bottom", "inner", "outer", "axial", "radial", "circumferential", etc. are based on the orientations or positions shown in the accompanying drawings. They are intended merely to facilitate and simplify the explanation of the invention and do not indicate or imply that the stated components or elements have to assume, or be constructed or operated in, particular orientations. Therefore, they are not to be construed as limiting the invention. In the description herein, unless otherwise specified, the meaning of "plurality" is two or more.

In the description herein, unless otherwise expressly specified and defined, the terms "mounting", "coupling", "connection" and "securing" should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context.

In the description herein, unless otherwise expressly specified and defined, when a first feature is described as being "on" or "under" a second feature, it can be in direct contact with the second feature, or intervening feature(s) may also be present. Moreover, when a first feature is described as being "over", "overlying" or "above" a second feature, it may either be situated normally or obliquely over the second feature, or it may only be located at a horizontal height higher than a horizontal height at which the second feature is located. When a first feature is described as being "under", "underlying" or "beneath" a second feature, it may either be situated normally or obliquely under the second feature, or it may only be located at a horizontal height lower than a horizontal height at which the second feature is located.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A connection device, comprising a plurality of first connectors, a casing, a pressing member and a fastening assembly, wherein
the plurality of the first connectors, the pressing member and the fastening assembly are at least partially housed in the casing, the pressing member has a pressing portion which is exposed out of the casing and configured to drive the fastening assembly, the first connectors are configured to connect with second connectors,
the fastening assembly is configured to switch between a ready-to-fasten configuration and a fastening configuration, so as to change connection conditions between the first connectors and the second connectors.

2. The connection device according to claim 1, wherein the fastening assembly comprises a fastener, a first elastic member, a push pin and a second elastic member, the fastener movably arranged at the first connectors, the first elastic member abutting, at opposite ends thereof, against the fastener and the first connector and configured to drive the fastener to move up and down, the second elastic member abutting, at opposite ends thereof, against the push pin and the first connector and configured to drive the push pin to move horizontally.

3. The connection device according to claim 2, wherein the ready-to-fasten configuration of the connection device results from pressing the pressing portion to cause the fastener to move over an end surface of the first connector from a first position to a second position where the fastener is engaged by the push pin, and
wherein the fastening configuration of the connection device results from inserting a respective one of the second connectors into the first connector so that the second connector abuts against the push pin and disengages the fastener from the push pin, as a result of which the fastener fastens the second connector.

4. The connection device according to claim 2 or 3, wherein the fastener is a bent plate comprising a first plate portion and a second plate portion extending from an end of the first plate portion, the first plate portion connected to the pressing member and the first elastic member so that the first elastic member abuts, at opposite ends thereof, against the first plate portion and the first connector, the second plate portion arranged at an end of the first connector.

5. The connection device according to claim 4, wherein the second plate portion defines a first opening and a second opening, the first opening is configured for passage of the second connector therethrough, the second opening is configured for passage of the push pin therethrough, the first opening matches with a groove in the second connector, and the first opening is able to engage with the groove to fasten the second connector.

6. The connection device according to claim 4, wherein the pressing member defines, at an end thereof, a receptacle pocket in which the first plate portion is inserted, and the pressing member is, at a further end thereof, pivotally coupled to the casing.

7. The connection device according to any one of claims 4 to 6, wherein the first connector is provided, on an end surface thereof, with guide features in which the second plate portion is arranged so as to move up and down over the end surface of the first connector to change a configuration of the fastening assembly.

8. The connection device according to any one of claims 5 to 7, wherein the push pin is cylindrical and comprises a first cylindrical section and a second cylindrical section, which are joined to each other, the first cylindrical section having a width that is less than a width of the second cylindrical section, the first cylindrical section configured to engage the fastener, the second cylindrical section configured to abut against the second elastic member.

9. The connection device according to claim 8, wherein the first cylindrical section defines, at middle thereof, an annular groove, and wherein the second opening comprises a first opening portion and a second opening portion, which are joined to each other, the first opening portion configured for passage of the first cylindrical section therethrough, the second opening portion configured to engage the annular groove.

10. The connection device according to any one of claims 2 to 9, wherein the first connector defines a first accommodating recess, and wherein the first elastic member has an end received in the first accommodating recess and a further end abutting against the fastener.

11. The connection device according to any one of claims 2 to 10, wherein the first connector defines a second accommodating recess, and wherein the second elastic member has an end received in the second accommodating recess and a furhter end abutting against the push pin.

12. The connection device according to any one of claims 1 to 11, wherein the casing comprises an upper shell and a lower shell, the upper shell defining a through hole through which the pressing portion is exposed out of the casing.

13. The connection device according to any one of claims 1 to 12, wherein the casing defines a plurality of accommodating spaces for locating the respective first connectors, and wherein the single fastening assembly is disposed in correspondence with one of the accommodating spaces so that the single fastening assembly can be manipulated to simultaneously change connection conditions between the first connections and the second connectors.

14. The connection device according to claim 13, wherein the fastening assembly is disposed in correspondence with a middle one of the accommodating spaces.

15. A connector, comprising second connectors and the connection device according to any one of claims 1 to 14.

16. A heat exchange device, comprising the connector according to claim 14 and at least one of a fluidic connection pipe configured to be connected to the connector, a heat exchange and pressurization bag and a heat exchange and pressurization apparatus.
